# EUROPEAN PATENT SPECIFICATION

(11) **EP 3 972 568 B1**
(45) Date of publication and mention of the grant of the patent: **03.12.2025**
(21) Application number: 20731402.2
(22) Date of filing: 22.05.2020
(51) Int. Cl.: A61K 9/48, A61K 31/375, A23L 33/15, A23L 33/155, A61K 47/12

(54) **VITAMIN AND MINERAL SOFTGEL CAPSULE PREPARATIONS COMPRISING VITAMIN C IN THE FORM OF AN ASCORBATE SALT**
VITAMIN- UND MINERAL-SOFTGELKAPSELPRÄPARATE MIT VITAMIN C IN FORM EINES ASCORBATSALZES
PRÉPARATIONS DE CAPSULES DE GEL MOU DE VITAMINES ET DE MINÉRALES COMPRENANT DE LA VITAMINE C SOUS FORME D'UN SEL D'ASCORBATE

(30) Priority: 22.05.2019 EP 19382412
(43) Date of publication of application: 30.03.2022
(73) Proprietor: Bayer Consumer Care AG, 4052 Basel (CH)
(72) Inventor: ZUMETA PÉREZ, Javier, 28806 Alcalá de Henares (ES); SANZ SAIZ, María del Pilar, 28806 Alcalá de Henares (ES); CABALLO GONZÁLEZ, María Angela, 28806 Alcalá de Henares (ES); IGLESIAS PINEIRO, María Elena, 28806 Alcalá de Henares (ES); TENA SERRANO, Rubén, 28806 Alcalá de Henares (ES); MONTES OVIANO, Lucía, 28806 Alcalá de Henares (ES); PRIOR CABANILLAS, Alberto, 28806 Alcalá de Henares (ES)
(74) Representative: BIP Patents
(86) International application number: PCT/EP2020/064288
(87) International publication number: WO 2020/234458

(56) References cited:
- US-A1- 2010 272 792
- US-A1- 2012 100 123
- US-B1- 9 629 846
- RITA R ELLITHORPE ET AL: "Pilot Study: Reduction of Fatigue by Use of a Dietary Supplement Containing Glycophospholipids", THE JOURNAL OF THE AMERICAN NUTRACEUTICAL ASSOCIATION, vol. 6, no. 1, 1 January 2003 (2003-01-01), pages 23 - 28, XP055151750
- ANONYMOUS: "Supplemental Forms |The Bioavailability of Different Forms of Vitamin C (Ascorbic Acid)", 3 August 2016 (2016-08-03), pages 1 - 10, XP055638502, Retrieved from the Internet <URL:https://lpi.oregonstate.edu/mic/vitamins/vitamin-C/supplemental-forms> [retrieved on 20191104]

## Description

### Technical field

The invention relates to method of reducing the fragility pharmaceutical or dietary unit dosage softgel capsules, consisting essentially of vitamins wherein Ascorbic acid has been replaced by Calcium ascorbate or Magnesium ascorbate, and optionally minerals compounds and a pharmaceutically or dietetically suitable carrier such as Docosahexaoenoic acid omega 3 (DHA) and/or Eicosapentaenoic acid omega 3 (EPA).

### Background of the invention

The use of softgel capsules is increasing across a wide range of applications including prescription medicines, consumer health, vitamins, and mineral supplements.

The method for manufacturing a softgel product occurs in five stages: preparation of the shell mass, manufacturing of fill material, encapsulation process, drying, and finishing (see, e.g., Hutchison KG, Ferdinando J. "Soft capsules". In: Aulton ME, Taylor KMG. Aulton's pharmaceutics: the design and manufacture of medicine. Elsevier Health Sciences 2013, pp 597-610).

Traditionally, the outer shell (the "shell") is prepared from gelatin, plasticizer(s) and water. Optional materials could be included into the shell formula such as opacifiers, colorants, flavors, sweeteners and preservatives. This material is shaped into the shell that forms the outermost layer of the capsule and holds the internal fill material, which typically contains the API(s) or nutrient(s) and the excipient(s) that are used to fill the shell itself (the "fill").

One of the common challenges associated with softgel capsule performance is the tendency of the capsules to be brittle, especially in softgel gelatine multivitamin / multimineral capsules. Capsule manufacturers often associate such poor performance of a capsule to manufacturing processes or storage conditions. However, the composition of the shell or fill formulation could also play a role in this phenomena.

The key to preventing brittleness is often related to maintaining the optimal malleability in the capsule shell, although other factors, such as filling pressure and impacts during packaging, storing and/or transporting processes, also play a role.

The elasticity and malleability of the shell material is often related to the type of gelatin -origin, type of denaturing method, molecular weight, bloom strength-, type and quantity of plasticizer and pigment's content such as titanium dioxide's content, wherein it is known that high levels of titanium dioxide are often associated with a higher risk of brittleness.

Furthermore, the elasticity and malleability of the shell material is also related to the moisture content of the capsule shell. In this sense and in a constant manner, the water of the shell formula migrates from the shell material to the environment. Softgels newly formed at the encapsulating machine show moisture content between 35 - 40% (weight by weight; w/w). During the drying process, the air penetrates the shell moving the water outward to the softgel surface reducing the initial water content to 20 - 25% (w/w) in the first or dynamic drying process and to 10 - 15% (w/w) in the second or static drying process (see, e.g., Gullapalli RP. Soft gelatin capsules (softgels). J Pharm Sci. 2010; 99(10): 4107-48), wherein if the capsules lose an excessive moisture (over-dried), they tend to become brittle. This phenomenon also takes place during storage. In order to minimize water migration, factors such as the gelatin age, gelatin ribbon thickness and the lubricant used during the encapsulation process could be optimized, and the drying and storing conditions (temperature, humidity and air flow conditions) could be further controlled. Indeed, during cold winter months, heating systems dry-out the air to 15 to 20 percent relative humidity. Capsules openly exposed to such dry air will lose moisture in a matter of minutes, becoming brittle. In these conditions, portable industrial humidifiers can be used to maintain relative humidity at an optimal range of 45 to 50 percent. Using re-sealable bag ties for opened capsule cartons will make it easier for operators to protect capsules while in the encapsulation room. Therefore, it is clear that manufacturing and storage conditions shall affect the brittleness of the softgel capsules.

Moreover, the free water of the shell formula can migrate to the fill. The presence of hygroscopic compounds into the filling, which attract water from its surroundings through either absorption or adsorption, could promote brittle capsules by decreasing the elasticity of the shell material or increasing internal pressure. Consequently, it is important to realize that formulation parameters also play an important role in the overall capsule performance, including its tendency to become brittle.

In conclusion, numerous formulation parameters can affect the mechanical properties of raw materials and the capsule itself. Extensive experience in the field of capsule manufacturing is needed to identify core fundamental properties that affect capsule performance. Therefore, it is important for manufacturers to also be cognizant of and have a stringent check over these parameters to improve the soft gelatin capsule performance.

The present invention confronts the technical problem of identifying further formulation parameters that have a considerable impact on the brittleness of softgel capsules and that resolve this problem.

Ellithorpe R.R. et al., J Am Nutraceutical Ass. 6(1) (2003) pp. 23-28 discloses formulations of tablets and softgel capsules comprising calcium ascorbate, EPA, DHA, vitamins such as vit. A, D3, E, K, B1, B2, B3, B6, B12, as well as folic acid, biotin, pantothenic acid, minerals such as magnesium, zinc, selenium, copper, manganese, chromium, molybdenum and further nutrients.

US 2012/100123 A1 discloses softgel formulations comprising ascorbate, DHA, EPA, vitamins and minerals, also in capsules.

US 2010/272792 A1 discloses tablets or powder filled capsules comprising calcium ascorbate, DHA, EPA, vitamins and minerals, such as vitamins D3, E, B5, folic acid, vitamin B 12, iron, copper, and magnesium.

US 9 629 846 81 discloses nutritional supplement for women comprising vitamin C as sodium ascorbate, DHA, EPA and further vitamins and minerals.

### BRIEF DESCRIPTION OF THE FIGURE

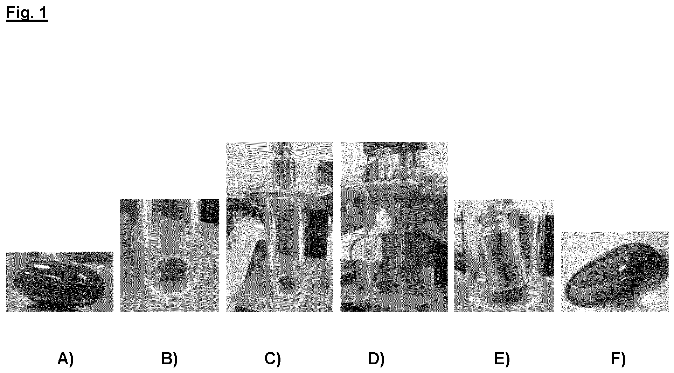

**Figure 1****.** These figures illustrate the internal test used in the examples to evaluate the brittleness of soft gelatin capsules. The method consists on placing each capsule on a metal test platform in such a way that the seam is always parallel to the plate (Figure 1A). A Methacrylate tube is positioned in such a way that the capsule is centered in the tube (Figure 1B). A weight is disposed on the platform and placed on its top part (Figure 1C) and then the platform is opened for the weight to fall on the capsule (Figure 1D), after the fall of the weight the integrity of the capsule is checked (Figure 1E and Figure 1F).

### DESCRIPTION OF THE INVENTION

The present invention may be understood more readily by reference to the following detailed description of the preferred embodiments of the invention, and to the example included therein.

As used herein, the term "pharmaceutical composition" means a composition, which is suitable for prescription and OTC medicaments, and which are available from doctors, in chemist's shop or in drugstores, only.

As used herein, the term "dietary composition " means a composition, which is for supplementing the regular food intake with additional nutritional elements to enhance quality of life, and which are freely available without prescription in groceries or super market, but not only in drugstores.

As used herein, the pharmaceutical or dietary composition is formulated in the form of softgel gelatin capsules.

The present invention is confronted with the problem of identifying a potential root cause of general fragility issues in softgel gelatine multivitamin / multimineral capsules and to identify a solution to this problem.

Interestingly, as shown in example 1, fragility tests conducted with standard multivitamin / multimineral formulations and with different batches derived therefrom, free of Vitamin C or containing Vitamin C in the form of Ascorbic acid or in the form of an ascorbate salt such as Calcium ascorbate, indicated that the fragility of these standard products could be avoided by eliminating Vitamin C from the formulation or by replacing Ascorbic acid with a source of Vitamin C in the form of an ascorbate salt such as Calcium ascorbate. None of the other substitutions tested and shown in this example, had a real and lasting impact on the fragility of such compositions tested. Furthermore, any of the changes made related to the shell formula (gelatin type, plasticizer type and plasticizer amount), encapsulation process parameters (gelatin ribbon thickness and type of lubricant) and drying conditions (air flow, temperature and humidity) resolve the brittleness issue of these products.In addition, the experiments performed and detailed in example 2 of the present specification concluded that the removal of Vitamin C in the form of Ascorbic acid from the fill formulation of softgel capsules avoided the fragility problems associated to multivitamin derived products. None of the other substitutions tested and shown in the example, had a real and lasting impact on the fragility of such compositions tested.

Consequently, the results as provided herein indicate that Ascorbic acid interacts with other components of the filling, weakening the shell, and giving rise to fragile capsules. The use of Vitamin C in the form of an Ascorbate salt minimizes these interactions avoiding the damage in the envelope and its consequent fragility.

Therefore, a first aspect of the present disclosure, which is not part of the claimed invention, provides a composition, preferably a dietary or pharmaceutical composition, comprising or consisting of vitamin(s) (a), and optionally minerals (b), and a pharmaceutically or dietetically suitable carrier such as nutrients DHA and/or EPA (c), encapsulated in a soft gelatine such as a bovine, porcine, vegetable and succinylated gelatine shell, wherein the vitamin(s) include Vitamin C and said Vitamin C is, at least in part, in the form of an ascorbate salt such as, but not limited to, Calcium ascorbate or any other divalent cation salt such as Magnesium ascorbate.

In particular, the first aspect of the present disclosure, provides a composition, preferably a dietary or pharmaceutical composition, comprising or consisting of vitamin(s) (a), and optionally minerals (b), and a pharmaceutically or dietetically suitable carrier such as nutrients DHA and/or EPA (c), encapsulated in a soft gelatine, wherein the raw material used to provide the Vitamin C in the final dietary or pharmaceutical composition is in the form of an ascorbate salt such as, but not limited to, Calcium ascorbate or any other divalent cation salt such as Magnesium ascorbate and not in the form of Ascorbic acid. In this sense, it is noted that, in the present disclosure, the original source, the raw material, used to introduce Vitamin C in the final dietary or pharmaceutical composition must be at least in part an ascorbate salt such as, but not limited to, Calcium ascorbate or any other divalent cation salt such as Magnesium ascorbate. Preferably, the original source for the vitamin C present in the final dietary or pharmaceutical composition must be at least in a 10% (w/w) an ascorbate salt such as, but not limited to, Calcium ascorbate or any other divalent cation salt such as Magnesium ascorbate, wherein such percentage by weight is calculated by determining the amount of the ascorbate salt over the total amount of the sum of the ascorbate salt and the Ascorbic acid present in the raw material used for introducing Vitamin C present in the final dietary or pharmaceutical composition. More preferably, the original source, raw material, for the Vitamin C present in the final dietary or pharmaceutical composition must be at least in a 20%, 30%, 40%, 50%, 60%, 70%, 80%, 90%, 95%, 96%, 97%, 98%, or 99% (w/w) an ascorbate salt such as, but not limited to, Calcium ascorbate or any other divalent cation salt such as Magnesium ascorbate, wherein such percentages by weight are calculated by determining the amount of the ascorbate salt over the total amount of the sum of the ascorbate salt and the ascorbic acid present in the raw material used for introducing Vitamin C present in the final dietary or pharmaceutical composition. Still more preferably, the original source, raw material, used for introducing Vitamin C in the final dietary or pharmaceutical composition is a substantially pure, more than 80%, still more preferably more than 90%, 95%, 96%, 97%, 98%, or 99% pure ascorbate salt such, but not limited to, as Calcium ascorbate or any other divalent cation salt such as Magnesium ascorbate.

Calcium ascorbate is a compound with the molecular formula CaC₁₂H₁₄O₁₂. It is the Calcium salt of Ascorbic acid, one of the mineral ascorbates. It is approximately 10% Calcium by mass. As a food additive, it has the E number E 302. It is approved for use as a food additive in the EU, USA and Australia and New Zealand.

Consequently, the compositions of the first aspect of the inventiondisclosure, comprise Vitamin C and may further comprise other vitamins, minerals or other type of nutrients, as long as the raw material used to produce the Vitamin C present in the final dietary or pharmaceutical composition, or the Vitamin C present in the final dietary or pharmaceutical composition, is substantially present in the form of an ascorbate salt (as defined above) and wherein such dietary or pharmaceutical composition might further consists or comprise at least one or any mixtures thereof of, for example, Vitamin A (i.e. Retinol palmitate or β-Carotene), Vitamin B1 (i.e. Thiamin or Thiamin mononitrate), Vitamin B2 (i.e. Riboflavin), Vitamin B3 (i.e. Nicotinamide), Vitamin B5 (i.e. Pantothenic acid or Calcium pantothenate), Vitamin B6 (i.e. Pyridoxine or Pyridoxine hydrochloride), Vitamin B9 (i.e Folic acid or Metafolin), Vitamin B12 (i.e Cyanocobalamin), Vitamin H (i.e Biotin), Vitamin D (i.e Vitamin D3), Vitamin E (i.e DL-α-Tocopheryl acetate, DL-α-Tocopherol or natural D-α-Tocopherol), Vitamin K, Choline ( i.e. Bitartrate choline), Iron (i.e. Carbonyl iron, Ferrous fumarate or Ferrous sulphate), Iodine (i.e. Potassium iodide or Potassium iodate), Magnesium (i.e. Magnesium oxide), Zinc (i.e. Zinc oxide or Zinc sulphate), Selenium (i.e. Sodium selenate), Copper (i.e. Cupric oxide or Copper sulphate), Manganese (i.e. Manganese sulfate), Calcium (i.e. Calcium phosphate or Calcium carbonate), Chromium (i.e. Chromium chloride or Chromium picolinate), Molybdenum (i.e. Sodium molybdate or Ammonium molybdate), Fluoride (i.e. Calcium fluoride), Chloride, Potassium, Sodium, DHA (i.e. DHA fish oil Ethyl ester, DHA fish oil Triglycerides or DHA algae), and/or EPA (i.e. DHA fish oil Ethyl ester, DHA fish oil Triglycerides or DHA algae). Such vitamins, minerals and other nutrients are commercially available from sources known by those skills in the art.

Preferably the dietary or pharmaceutical composition according to the disclosure consists or comprises Vitamin C in the form of an ascorbate salt such as, but not limited to, Calcium ascorbate or any other divalent cation salt such as Magnesium ascorbate (as described above), and at least one further vitamin selected from the group comprising or consisting of Vitamin A (such as β-Carotene), Vitamin B1 (such as a Thiamin salt), Vitamin B2 (such as Riboflavin), Vitamin B3 (such as Nicotinamide), Vitamin B5 (such as a Pantothenate salt), Vitamin B6 (such as a Pyridoxine salt), Vitamin B12 (such as Cyanocobalamin), Vitamin D (such as Colecalciferol), Vitamin E (such as α-Tocopheryl acetate), Vitamin B9 (such as Folic acid or Metafolin acid), and Vitamin H (such as Biotin), or any combination thereof.

Also preferably, the pharmaceutical or dietary composition according to the disclosure, consists of or comprises vitamins (a) selected from Vitamin C in the form of an ascorbate salt such as, but not limited to, Calcium ascorbate or any other divalent cation salt such as Magnesium ascorbate, and at least one further vitamin selected from the group consisting of or comprising Vitamin A (such as Retinol palmitate and β-Carotene), Vitamin B1 (such as a Thiamin salt), Vitamin B2 (such as Riboflavin), Vitamin B3 (such as Nicotinamide), Vitamin B5 (such as a Pantothenate salt), Vitamin B6 (such as a Pyridoxine salt), Vitamin B12 (such as Cyanocobalamin), Vitamin D (such as Colecalciferol), Vitamin E (such as DL-α-Tocopheryl acetate, DL-α-Tocopherol or natural D-α-Tocopherol), Vitamin B9 (such as Folic acid), and Vitamin H (such as Biotin), or any combination thereof.

In a further preferred embodiment of the first aspect of the disclosure, the pharmaceutical or dietary composition comprises one or more minerals or nutrients selected from at least one of the list consisting or comprising of Iron (such as Ferrous fumarate), Iodide (preferably as Potassium iodate), Magnesium (preferably as Magnesium oxide), Zinc (preferably as Zinc oxide), Selenite (such as Sodium selenite) and Copper (preferably as Sulphate copper), or any combination thereof.

In a still further preferred embodiment of the first aspect of the disclosure, the pharmaceutical or dietary composition comprises minerals selected from the list consisting of or comprising Iron (as Carbonyl iron), Iodide (preferably as Potassium iodide), Calcium (preferably as Calcium carbonate), Magnesium (preferably as Magnesium oxide), Zinc (preferably as Zinc oxide), and Copper (preferably as Copper oxide), or any combination thereof.

More preferably the dietary or pharmaceutical composition according to the disclosure comprises Vitamin C in the form of an ascorbate salt such as, but not limited to, Calcium ascorbate or any other divalent cation salt such as Magnesium ascorbate, and at least one further vitamin selected from the group comprising or consisting of Vitamin A (such as β-Carotene), Vitamin B1 (such as a Thiamin salt), Vitamin B2 (such as Riboflavin), Vitamin B3 (such as Nicotinamide), Vitamin B5 (such as Pantothenate salt), Vitamin B6 (such as a Pyridoxine salt), Vitamin B12 (such as Cyanocobalamin), Vitamin D (such as Colecalciferol), Vitamin E (such as α-Tocopheryl acetate), Vitamin B9 (such as Folic acid or Metafolin acid), and Vitamin H (such as Biotin); and wherein such pharmaceutical or dietary composition further comprises minerals selected from the list consisting or comprising of Iron (as Ferrous fumarate), Iodide (preferably as Potassium iodate), Magnesium (preferably as Magnesium oxide), Zinc (preferably as Zinc oxide), Selenite (such as Sodium selenite) and Copper (preferably as Sulphate copper), or any combination thereof.

Also preferably, in another preferred embodiment of the first aspect of the disclosure, the pharmaceutical or dietary composition consists or comprises vitamins (a) selected from Vitamin C in the form of an ascorbate salt such as, but not limited to, Calcium ascorbate or any other divalent cation salt such as Magnesium ascorbate, and at least one further vitamin selected from the group consisting of or comprising Vitamin A (such as Retinol palmitate and β-Carotene), Vitamin B1 (such as a Thiamin salt), Vitamin B2 (such as Riboflavin), Vitamin B3 (such as Nicotinamide), Vitamin B5 (such as a Pantothenate salt), Vitamin B6 (such as a Pyridoxine salt), Vitamin B12 (such as Cyanocobalamin), Vitamin D (such as Colecalciferol), Vitamin E (such as DL-α-Tocopheryl acetate, DL-α-Tocopherol or natural D-α-Tocopherol), Vitamin B9 (such as Folic acid), and Vitamin H (such as Biotin); and wherein such pharmaceutical or dietary composition further comprises minerals selected from the list consisting of or comprising Iron (as Carbonyl iron), Iodide (preferably as Potassium iodide), Calcium (preferably as Calcium carbonate), Magnesium (preferably as Magnesium oxide), Zinc (preferably as Zinc oxide), and Copper (preferably as Sulphate copper and/or Copper oxide), or any combination thereof.

Therefore, a wide variety of vitamins, and minerals that are preferably safe for consumption by any person having a deficiency in minerals and/or vitamins such as, but not limited to, pregnant women, lactating women or women having childbearing potential that are attempting to become pregnant may be used in the dosage form of the disclosure in varying quantities, as long as the vitamin(s) include Vitamin C, and the raw material used to produce the Vitamin C present in the dietary or pharmaceutical composition, or the Vitamin C present in the final dietary or pharmaceutical composition, is in the form of an ascorbate salt such as, but not limited to, Calcium ascorbate or any other divalent cation salt such as Magnesium ascorbate. It is noted that a wide variety of fats and oils can be employed as carriers of the nutrients of the dietary or pharmaceutical composition according to the disclosure. These fats or oils include, for example, olive oil, canola oil, palm oil, coconut oil, sunflower oil, peanut oil, vegetable oil, lecithin, fish oil, cotton seed oil, soybean oil, lard, monoglycerides, diglycerides, butter, margarine, and other animal, vegetable, and marine fats, and milk fats, waxes such as beeswax, which are commercially available from sources known by those of skill in the art, and mixtures thereof. Vegetable oil is the preferred fat for use in the food bars of the disclosure. Preferably, DHA (Docosahexaoenoic acid omega) or EPA can be employed. Moreover, these compounds act as nutrients. DHA is a long-chain fatty acid that is necessary for brain and eye development in children, and may be included as an ingredient of the dosage form of the disclosure in an amount ranging from about 10 to about 300 mg, with about 100 to 200 mg being preferred, and about 150 mg being most preferred for pregnant women, lactating women, and women having childbearing potential that are attempting to become pregnant. On the other hand, gelatine is an essential component of the soft gelatine shells of the instant disclosure. The starting gelatine material used in the manufacture of soft capsules is obtained by the partial hydrolysis of collagenous material, such as the skin, white connective tissues, or bones of animals. Depending on the thermal denaturing process used to obtain gelatin form collagen, gelatine material can be classified as Type A gelatine, which is obtained from the acid-processing of bovine bone, bovine hide and porcine skin exhibiting an isoelectric point between pH 7 and pH 9; and Type B gelatine, which is obtained from the alkaline-processing of bovine bone and bovine hide and exhibits an isoelectric point between pH 4.7 and pH 5.2. Blends of Type A and Type B gelatines can be used to obtain a gelatine with the requisite viscosity and bloom strength characteristics for capsule manufacture. Gelatine suitable for capsule manufacture is commercially available from the Sigma Chemical Company, St. Louis, Mo. For a general description of gelatine and gelatine-based capsules, (see, e.g., Remington's Pharmaceutical Sciences, 16th ed., Mack Publishing Company, Easton, Pa. (1980), page 1245 and pages 1576-1582; and U.S. Pat. No. 4,935,243, to Borkan et at., issued Jun. 19, 1990).

The soft gelatine shell of the capsules of the instant disclosure, as initially prepared, comprises from about 20% to about 60% (w/w) gelatine, more preferably from about 25% to about 50% (w/w) gelatine, and most preferably from about 40% to about 50% (w/w) gelatine. The gelatine can be of Type A & Type B, or a mixture thereof with bloom numbers ranging from about 60 to about 300.

A plasticizer is another component of the soft gelatine shells of the instant disclosure. One or more plasticizers are incorporated to produce a soft gelatine shell. The soft gelatine thus obtained has the required flexibility characteristics for use as an encapsulation agent. Useful plasticizers of the present disclosure include glycerine, sorbitan, sorbitol, or similar low molecular weight polyols, and mixtures thereof.

The shell of the present disclosure, as initially prepared, generally comprises from about 10% to about 35% (w/w) plasticizer, preferably from about 15% to about 30% (w/w) plasticizer, and most preferably from about 20% to about 30% (w/w) plasticizer. A preferred plasticizer useful in the present disclosure is glycerine.

The soft gelatine' shells of the instant disclosure also comprise water. Without being limited by theory, the water is believed to aid in the rapid dissolution or rupture of the soft gelatine shell upon contact with the gastrointestinal fluids encountered in the body.

The shell of the present disclosure, as initially prepared, generally comprises from about 15% to about 50% (w/w) water, more preferably from about 25% to about 40% (w/w) water, and most preferably from about 30% to about 40% (w/w) water.

Other optional components which can be incorporated into the soft gelatin shells include colorings including color coatings, flavorings, preservatives, anti-oxidants, essences, and other aesthetically pleasing components.

The compositions of the present disclosure can be encapsulated within any conventional soft gelatine shell that is capable of substantially containing the composition for a reasonable period of time. The soft gelatine shells of the instant disclosure can be prepared by combining appropriate amounts of gelatine, water, plasticizer, and any optional components in a suitable vessel and agitating and/or stirring while heating to about 65 °C, until a uniform solution is obtained. This soft gelatine shell preparation can then be used for encapsulating the desired quantity of the fill composition employing standard encapsulation methodology to produce one-piece, hermetically-sealed, soft gelatine capsules. The gelatine capsules are formed into the desired shape and size so that they can be readily swallowed. The soft gelatine capsules of the instant disclosure are of a suitable size for easy swallowing and typically contain from about 100 mg to about 2000 mg of the active composition. Soft gelatine capsules and encapsulation methods are described in P. K. Wilkinson et at., "Softgels: Manufacturing Considerations", Drugs and the Pharmaceutical Sciences, 41 (Specialized Drug Delivery Systems), P. Tyle, Ed. (Marcel Dekker, Inc., New York, 1990) pp.409-449; F. S. Horn et at., "Capsules, Soft", Encyclopedia of Pharmaceutical Technology, vol. 2, J. Swarbrick and J. C. Boylan, eds. (Marcel Dekker, Inc., New York, 1990) pp. 269-284; M. S. Patel et at., "Advances in Softgel Formulation Technology", Manufacturing Chemist, vol. 60, no. 7, pp. 26-28 (July 1989); M. S. Patel et al., "Softgel Technology", Manufacturing Chemist, vol. 60, no. 8, pp. 47-49 (August 1989); R. F. Jimerson, "Softgel (Soft Gelatin Capsule) Update", Drug Development and Industrial Pharmacy (Interphex '86 Conference), vol. 12, no. 8 & 9, pp. 1133-1144 (1986); and W. R. Ebert, "Soft Elastic Gelatin Capsules: A Unique Dosage Form", Pharmaceutical Technology, vol. 1, no. 5, pp. 44-50 (1977);. The resulting soft gelatin capsule is soluble in water and in gatrointestinal fluids. Upon swallowing the capsule, the gelatin shell rapidly dissolves or ruptures in the gastrointestinal tract thereby introducing the pharmaceutical actives from the liquid core into the physiological system.

Preferably the capsules have an oblong or oval shape to facilitate swallowing. In the case of a capsule containing 300 to 700 mg of the combined active ingredients an oblong capsule may be about 10-28 mm, preferably 20-26 mm, in particular about 25 mm long and have a diameter of about 5 to 11 mm, preferably 6-10 mm, in particular 8-9 mm.

The composition according to the disclosure may preferably contain at least two, three, four, five or at least six nutrients selected from the group consisting of or comprising: Iron (i.e. Carbonyl iron, Ferrous fumarate or Ferrous sulphate), Iodide (i.e. Potassium iodide or iodate), Magnesium (i.e. Magnesium oxide), Calcium (i.e Calcium carbonate), Zinc (i.e. Zinc oxide or Zinc sulphate), Selenite Copper (i.e. Sulphate copper or Copper oxide), DHA and EPA. Furthermore, the composition according to the disclosure may preferably contain at least two, three, four, five or at least six vitamins selected from the group consisting of or comprising: Vitamin A (i.e. Retinol palmitate or β-Carotene), Vitamin B1 (i.e. Thiamin or Thiamin mononitrate), Vitamin B2 (i.e. Riboflavin), Vitamin B3 (i.e. Nicotinamide), Vitamin B5 (as Pantothenic Acid or Calcium pantothenate), Vitamin B6 (i.e Pyridoxine or Pyridoxine hydrochloride), Vitamin B9 (i.e. Folic acid or Metafolin), Vitamin B12 (i.e. Cyanocobalamin), Vitamin H (i.e. Biotin), Vitamin D (i.e. Vitamin D3), Vitamin E (i.e. DL-α-Tocopheryl acetate, DL-α-Tocopherol or D- α-Tocopherol), Choline (i.e. Bitartrate choline), and Vitamin K.

More particularly, the dietary or pharmaceutical composition according to the disclosure comprises +/- 200%, 150%, or 100%, preferably +/- 50%, more preferably +/- 40%, 35%, 30%, 25%, 20%, 15%, 10%, 5% or 1%, of the ingredients of formulas R21 and/or R22 below in mgs per capsule for the fill composition and percentage by weigh over the total components of the shell for the shell composition:

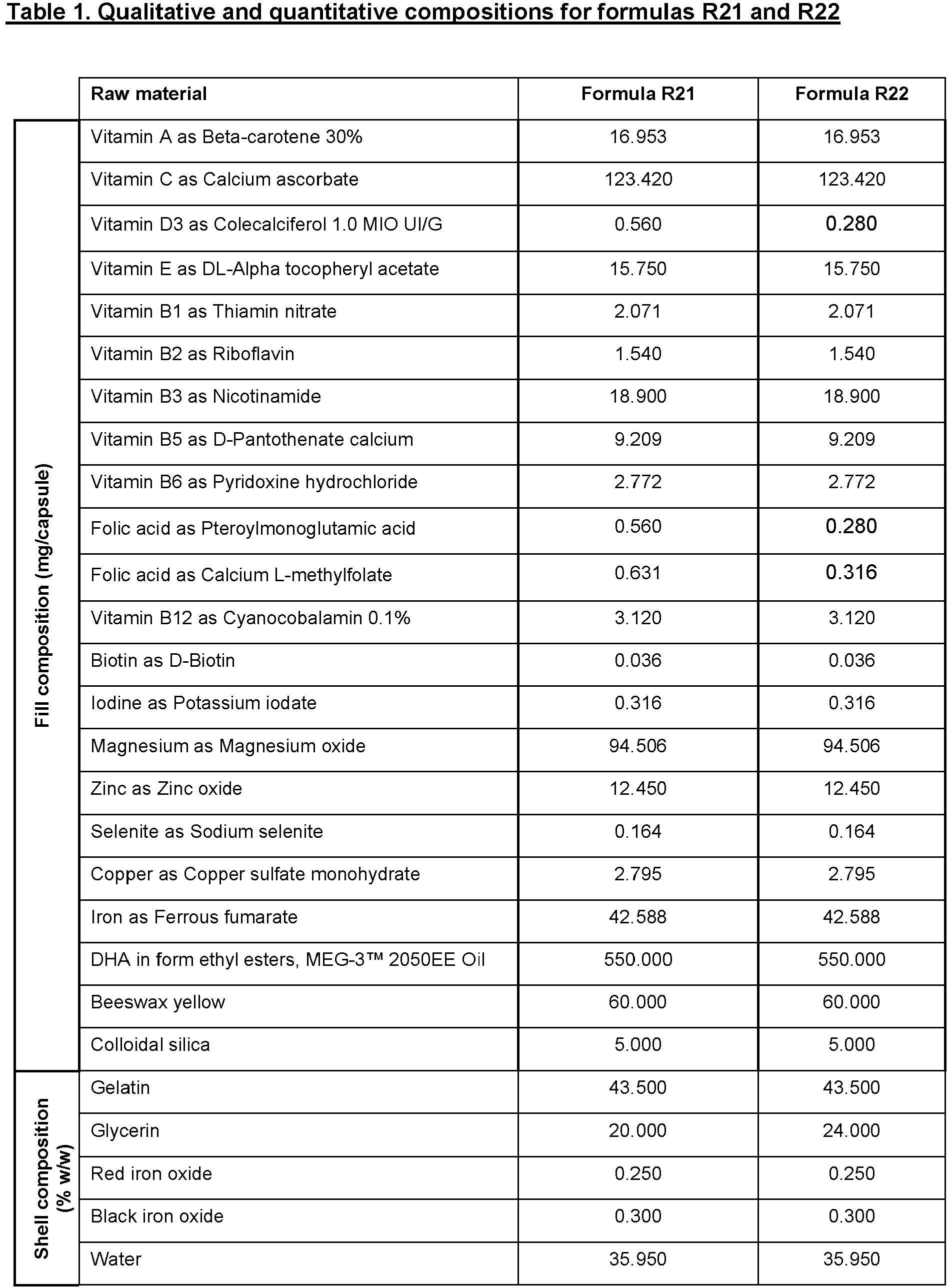

In another particular embodiment, the dietary or pharmaceutical composition according to the disclosure comprises +/- 200%, 150%, or 100%, preferably +/- 50%, more preferably +/- 40%, 35%, 30%, 25%, 20%, 15%, 10%, 5% or 1%, of the ingredients of each of formulas G3, G5, G7 or G8 below (in mg per capsule for the fill composition and percentage by weigh over the total components of the shell, for the shell composition):

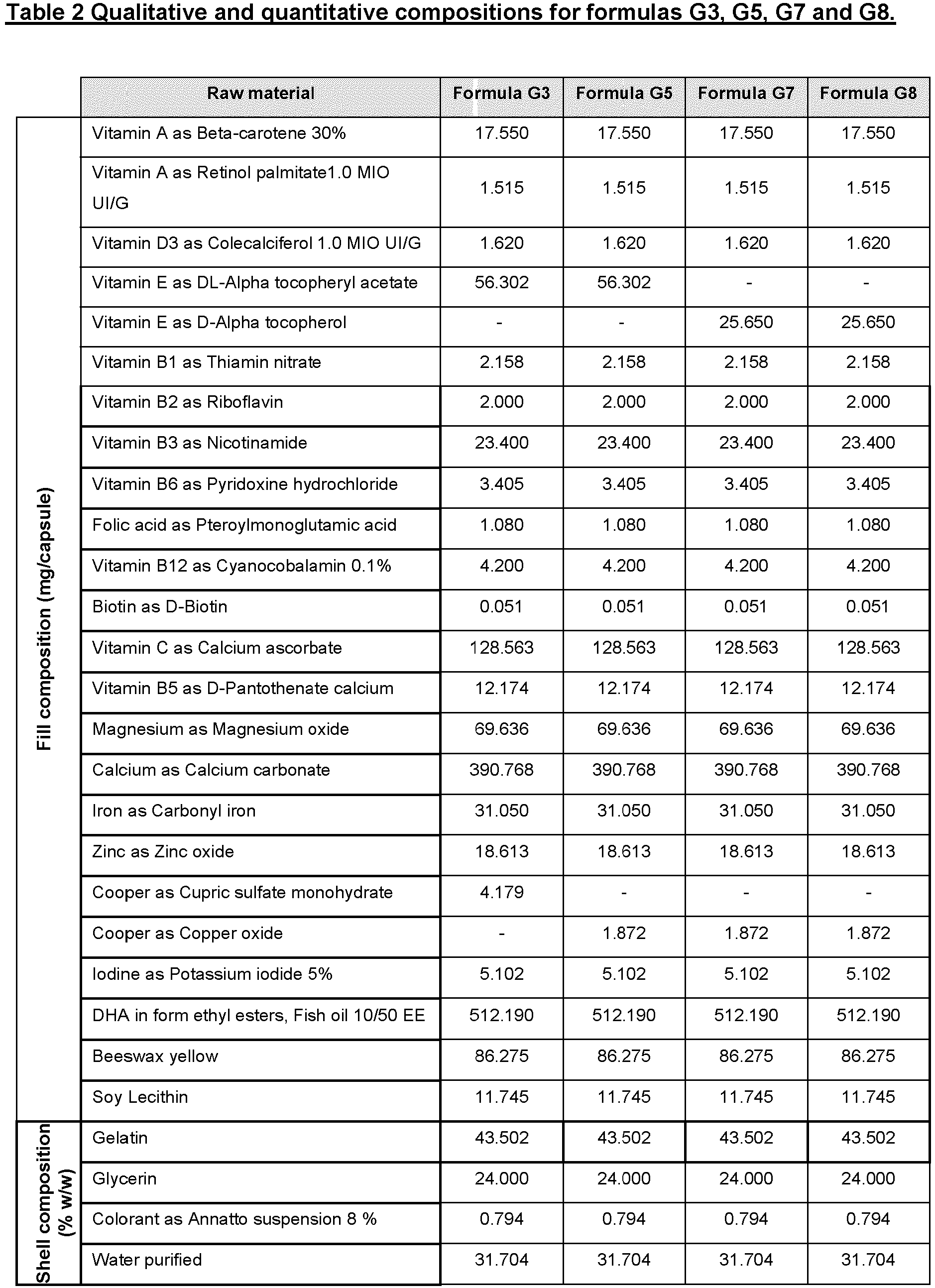

Any of the dosage forms of the disclosure, with the exception of the Vitamin C which must be formulated in the form of an ascorbate salt, at least in part, may be formulated using any pharmaceutically- acceptable forms of the vitamins and/or minerals described above, including their salts, which are known by those of skill in the art. For example, useful pharmaceutically-acceptable Magnesium compounds include Magnesium stearate, Magnesium carbonate, Magnesium oxide, Magnesium hydroxide and Magnesium sulfate. Pharmaceutically-acceptable Iron compounds include any of the well-known Iron II (ferrous) or Iron III (ferric) supplements, such as Ferrous sulfate, Ferric chloride, Ferrous gluconate, Ferrous lactate, Ferrous tartrate, Iron-sugar-carboxylate complexes, Ferrous fumarate, Ferrous succinate, Ferrous glutamate, Ferrous citrate, Ferrous pyrophosphate, Ferrous cholinisocitrate, Ferrous carbonate, and the like.

The vitamins and/or minerals that are employed in the softgel capsules of the disclosure are those that are preferably recommended for consumption by any person having a deficiency in minerals and/or vitamins such as, but not limited to, pregnant women, lactating women or women having childbearing potential that are attempting to become pregnant. These vitamins and minerals are employed in an amount that is effective for enhancing the nutrition of such person/s having a deficiency in minerals and/or vitamins such as, but not limited to, pregnant women, lactating women or women having childbearing potential that are attempting to become pregnant, or of their developing fetuses or babies.

Each dosage form may contain one or more of the above vitamins, minerals or other nutrients in any quantity that is safe for consumption by any person having a deficiency in minerals and/or vitamins such as, but not limited to, pregnant women, lactating women or women having childbearing potential that are attempting to become pregnant (i.e., a quantity that would not cause harm to the woman consuming the food bar, or to her developing fetus or breast-feeding baby).

As already stated, preferred are dosage forms according to the disclosure consisting essentially of (a) a multi- vitamin mixture consisting of lots based on formulas R21, R22, G3, G5, G7 or G8, which have Calcium ascorbate in the filling instead of Vitamin C in the form of Ascorbic acid, and were not deemed fragile. It is noted that such multi-vitamin mixture can contain about the same qualitative and quantitative compositions as shown for any of these dietary or pharmaceutical compositions.

A second aspect of the disclosure, which is not part of the claimed invention refers to a method of supplementing the dietary needs of any person having a deficiency in minerals and/or vitamins such as, but not limited to, pregnant woman, a lactating woman or a woman of childbearing potential who is attempting to become pregnant, said method comprising administering to the person having a deficiency in minerals and/or vitamins such as, but not limited to, a dietary supplementing amount of a pharmaceutical or dietary composition according to the first aspect of the disclosure. Preferably, one unit dosage form of the pharmaceutical or dietary composition is administered to such a person having a deficiency in minerals and/or vitamins per day.

A third aspect of the disclosure, which is part of the claimed invention refers to a method of reducing the fragility of pharmaceutical or dietary compositions in the form of a softgel gelatine capsule, consisting of or comprising (a) one or more vitamin(s), wherein at least one of those vitamin(s) is Vitamin C in the form of Ascorbic acid, and optionally (b) one or more minerals, and (c) pharmaceutically or dietetically suitable carriers such as DHA and/or EPA; wherein the method comprises replacing partially or totally the content of Vitamin C in the form of Ascorbic acid by Calcium ascorbate or Magnesium ascorbate.

It is noted that any of the vitamin(s), mineral(s) and/or other nutrients indicated in the first aspect of the disclosure may also be present in the pharmaceutical or dietary compositions identified in the third aspect of the disclosure, which is part of the claimed. In a preferred embodiment, the pharmaceutical or dietary compositions identified in the third aspect of the disclosure, are dietary or pharmaceutical compositions comprising Vitamin C in the form of Ascorbic acid, and at least one further vitamin selected from the group comprising or consisting of Vitamin A( such as β-Carotene), Vitamin B1 (such as a Thiamin salt), Vitamin B2 (such as Riboflavin), Vitamin B3 (such as Nicotinamide), Vitamin B5 (such as Pantothenate salt), Vitamin B6 (such as a Pyridoxine salt), Vitamin B12 (such as Cyanocobalamin), Vitamin D (such as Colecalciferol), Vitamin E (such as α-Tocopheryl acetate), Vitamin B9 (such as Folic acid or Metafolin acid), and Vitamin H (such as Biotin). Also preferably, the pharmaceutical or dietary compositions identified in the third aspect of the disclosure, are dietary or pharmaceutical compositions comprising Vitamin C in the form of Ascorbic acid, and at least one further vitamin selected from the group comprising or consisting of Vitamin A (such as Retinol palmitate and β-Carotene, Vitamin B1 (such as a Thiamin salt), Vitamin B2 (such as Riboflavin), Vitamin B3 (such as Nicotinamide), Vitamin B5 (such as Pantothenate salt), Vitamin B6 (such as a Pyridoxine salt), Vitamin B12 (such as Cyanocobalamin), Vitamin D (such as Colecalciferol), Vitamin E (such as DL-α-Tocopheryl acetate, DL-α-Tocopherol or D-α-Tocopherol), Vitamin B9 (such as Folic acid), and Vitamin H (such as Biotin). Also preferably, the pharmaceutical or dietary compositions identified in the third aspect of the disclosure, are dietary or pharmaceutical compositions comprising Vitamin C in the form of Ascorbic acid, and at least one further vitamin selected from the group comprising or consisting of Vitamin A (such as β-Carotene), Vitamin B1 (such as a Thiamin salt), Vitamin B2 (such as Riboflavin), Vitamin B3 (such as Nicotinamide), Vitamin B5 (such as Pantothenate), Vitamin B6 (such as a Pyridoxine salt), Vitamin B12 (such as Cyanocobalamin), Vitamin D (such as Colecalciferol), Vitamin E (α-Tocopheryl acetate), Vitamin B9 (such as Folic acid or Metafolin acid), and Vitamin H (such as Biotin); and wherein such pharmaceutical or dietary composition further comprises minerals selected from the list consisting or comprising of Iron (as Ferrous fumarate), Iodide (preferably as Potassium iodate), Magnesium (preferably as Magnesium oxide), Zinc (preferably as Zinc oxide), Selenite (such as Sodium selenite) and Copper (preferably as Sulphate copper), or any combination thereof. Also preferably, the pharmaceutical or dietary compositions identified in the third aspect of the disclosure, are dietary or pharmaceutical compositions comprising Vitamin C in the form of Ascorbic acid, and at least one further vitamin selected from the group comprising or consisting of Vitamin A (such as Retinol palmitate and β-Carotene), Vitamin B1 (such as a Thiamin salt), Vitamin B2 (such as Riboflavin), Vitamin B3 (such as Nicotinamide), Vitamin B5 (such as Pantothenate salt), Vitamin B6 (such as a Pyridoxine salt), Vitamin B12 (such as Cyanocobalamin), Vitamin D (such as Colecalciferol), Vitamin E (such as DL-α-Tocopheryl acetate, DL-α-Tocopherol or D-α-Tocopherol), Vitamin B9 (such as Folic acid), and Vitamin H (such as Biotin); and wherein such pharmaceutical or dietary composition further comprises minerals selected from the list consisting of or comprising Iron (as Carbonyl iron), Iodide (preferably as Potassium iodide), Calcium (preferably as Calcium carbonate), Magnesium (preferably as Magnesium oxide), Zinc (preferably as Zinc oxide), and Copper (preferably as Sulphate copper and/or Copper oxide), or any combination thereof.

A fourth aspect of the disclosure, which is not part of the claimed invention, refers to a method of preparation or synthesis of a pharmaceutical or dietary composition according to the first aspect of the disclosure, suitable for supplementing the dietary needs of any person having a deficiency in minerals and/or vitamins such as, but not limited to, pregnant woman, a lactating woman or a woman of childbearing potential who is attempting to become pregnant. Preferably, said preparation is for the preparation of a single pharmaceutical or dietary unit dosage form, which can be administered once a day.

It is finally contemplated that any features described herein can optionally be combined with any of the embodiments of any method, medical use, kit and use of a kit of the disclosure; and any embodiment discussed in this specification can be implemented with respect to any of these. It will be understood that particular embodiments described herein are shown by way of illustration and not as limitations of the invention.

The use of the word "a" or "an" may mean "one," but it is also consistent with the meaning of "one or more," "at least one," and "one or more than one". The use of the term "another" may also refer to one or more. The use of the term "or" in the claims is used to mean "and/or" unless explicitly indicated to refer to alternatives only or the alternatives are mutually exclusive.

As used in this specification and claim(s), the words "comprising" (and any form of comprising, such as "comprise" and "comprises"), "having" (and any form of having, such as "have" and "has"), "including" (and any form of including, such as "includes" and "include") or "containing" (and any form of containing, such as "contains" and "contain") are inclusive or open-ended and do not exclude additional, unrecited elements or method steps. The term "comprises" also encompasses and expressly discloses the terms "consists of" and "consists essentially of". As used herein, the phrase "consisting essentially of" limits the scope of a claim to the specified materials or steps and those that do not materially affect the basic and novel characteristic(s) of the claimed invention. As used herein, the phrase "consisting of" excludes any element, step, or ingredient not specified in the claim except for, e.g., impurities ordinarily associated with the element or limitation.

The term "or combinations thereof" as used herein refers to all permutations and combinations of the listed items preceding the term. For example, "A, B, C, or combinations thereof" is intended to include at least one of: A, B, C, AB, AC, BC, or ABC, and if order is important in a particular context, also BA, CA, CB, CBA, BCA, ACB, BAC, or CAB. Continuing with this example, expressly included are combinations that contain repeats of one or more item or term, such as BB, AAA, AB, BBC, AAABCCCC, CBBAAA, CABABB, and so forth. The skilled artisan will understand that typically there is no limit on the number of items or terms in any combination, unless otherwise apparent from the context.

As used herein, words of approximation such as, without limitation, "about", "around", "approximately" refers to a condition that when so modified is understood to not necessarily be absolute or perfect but would be considered close enough to those of ordinary skill in the art to warrant designating the condition as being present. The extent to which the description may vary will depend on how great a change can be instituted and still have one of ordinary skilled in the art recognize the modified feature as still having the required characteristics and capabilities of the unmodified feature. In general, but subject to the preceding discussion, a numerical value herein that is modified by a word of approximation such as "about" may vary from the stated value by ±1, 2, 3, 4, 5, 6, 7, 8, 9, or 10%. Accordingly, the term "about" may mean the indicated value ± 5% of its value, preferably the indicated value ± 2% of its value, most preferably the term "about" means exactly the indicated value (± 0%).

### EXAMPLES

### Example 1. Evaluation of the physical properties over time of new formulas derived from multivitamin / multimineral standard products (STD1 and STD2)

The aim of this example was to find out possible root causes for the brittleness of multivitamin / multimineral standard products STD1 and STD 2, the qualitative and quantitative composition of each of these two products is identified in Table 4 below.

In this sense, it is noted that we prepared such products according to their standard preparation methods (STD) as described below:
- **Preparation of fill formulation:**
   The fill material is obtained by mixing oil and beeswax yellow at 60 - 65 °C. When all beeswax yellow is dissolved, the temperature is reduced to 45 °C. After, minerals are added and mixed. In a second step, the temperature is reduced to 30 °C and vitamin compounds are added. Then, the preparation is mixed, homogenized, de-aerated and sieved. The mix is stored in closed tank with nitrogen before encapsulation.
- **Preparation of shell formulation:**
   The gelatin mass is obtained by mixing and melting in vacuum purified water, plasticizer, gelatins and colorants iron oxides.
- **Encapsulation process:**
   The fill material and the gelatin mass are processed into soft gelatin capsules using an encapsulation machine. Mixture of medium chain triglycerides with lecithin is used for ribbon lubrication and as anti-sticking agent for the capsules.
- **Drying process:**
   Soft gelatin capsules are obtained then dried. Drying is initially performed using a rotary dryer. Final drying is on trays into static dryer.

We also prepared such products by using different methodologies of preparation. The reason for using different preparation methods is because is known that by adjusting encapsulation process parameters and controlling the drying and storing conditions, a reduction in the brittleness of softgel capsules can be achieved. In this sense and as reflected in Table 3 below, the following parameters were tested in relation to the standard preparation methods:
- Encapsulation process
   - Gelatin age: 12 hours and 72 hours
   - Gelatin ribbon thickness: 0.92, 0.95 and 1.00 mm
   - Internal lubricant: mixture of Medium chain triglyceride and Lecithin or Vaselin
- Drying process
   - Number of fans with air flow "on" in dynamic drier: 2 or 4 fans
   - Static drying conditions: 22 °C / 20 % HR or 27 °C / 10 % HR

In order to evaluate the brittleness of the soft gelatin capsules produced by the different methods, an internal test was developed. The method consisted on placing each capsule on a metal test platform in such a way that the seam was always parallel to the plate (Figure 1A), wherein the position of the capsule was deemed critical for the test since such position had a clear influence on the results. A Methacrylate tube, with an intern diameter of 30 mm, an external diameter of 40 mm and a height of 100 mm, was selected and positioned in such a way that the capsule was centered in the tube (Figure 1B). A weight of 100 g was disposed on the platform and placed on its top part (Figure 1C) and then the platform was opened for the weight to fall on the capsule (Figure 1D), after the fall of the weight the integrity of the capsule was checked (Figure 1E and Figure 1F).

The test was made on a representative sample (25 capsules) and the results were expressed as the percentage of broken capsules. The product was considered fragile when the percentage of broken capsules was more than 12 percent.

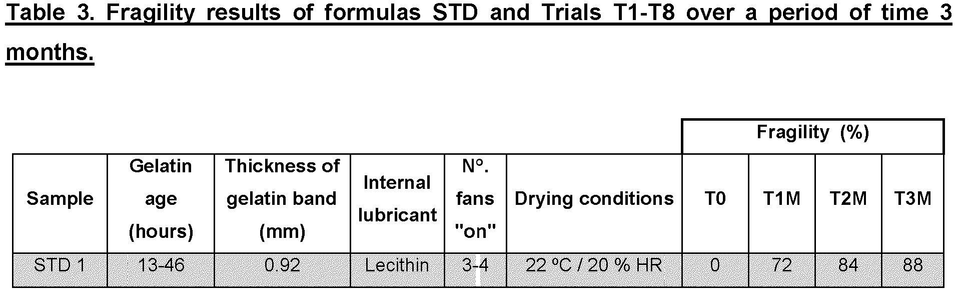

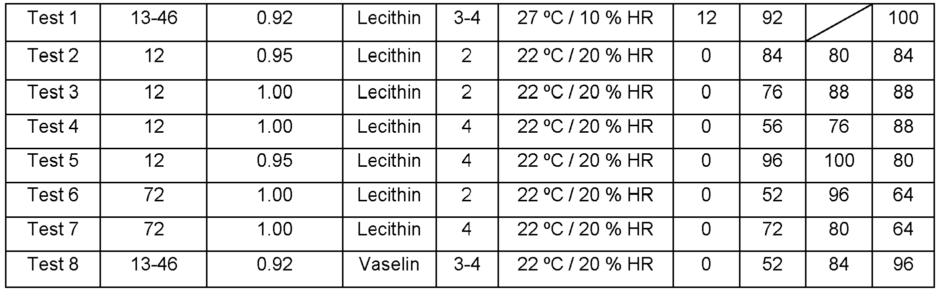

It is clear from the above results that the standard product, independently of their preparation method, resulted on the brittleness of the capsules (broken capsules ≥ 12 %).

Therefore, apart from evaluating the impact on the brittleness of the product of the different methodologies tested, we evaluated further causes for such fragility by preparing different qualitative and quantitative compositions derived from multivitamin / multimineral standard formulas (see compositions of formulations R1 to R22 in Table 4 and Table 5 below), including changes in shell formulation and fill formulation.

In this regard, as described in the literature, the type of gelatin -origin, type of denaturing method, molecular weight, bloom strength- can impact on the brittleness of a softgel capsule. Furthermore, depending on the type and quantity of plasticizer used, the fragility phenomena could be reduced.

On the other hand, we evaluated the effect of replacing different nutrients or excipients, which were found in the fill composition. In particular, we determined the presence or absence of Magnesium oxide, Tricalcium phosphate, Glycerin (at different concentrations), Lecithin, and Colloidal silica in the fill composition. We further evaluated the effect of replacing Ascorbic acid by Calcium ascorbate, Zinc sulphate by Zinc oxide, Ferrous sulphate by Ferrous fumarate and the type of DHA oils changing DHA fish oil Ethyl ester to DHA fish oil Triglycerides. Furthermore, different beeswax amounts were checked.

To comply with specifications about nutrient amount per capsule and size (13 oblong), fill weight per capsule was adjusted based on the physical parameters of each fill formulation.

Different finished product were packaged in polyethylene bags and stored at warehouse conditions (temperature between 15 - 25 °C).

In order to find the cause/s of brittleness, fragility of the different batches (formula STD and formulas R1 - R22) was further tested at different times (time zero, 1 month, 2 months 3 months, 6 months, 9 months and 12 months). The results were expressed as percentage of broken capsules and are summarized in Table 6 below.

To evaluate the fragility variable of each batch over time, Mood median test was used (95.0 % of CI). The values obtained show that the fragility of different products increases over time significantly (p-values ≤ 0.05) except to formulas R20, R21 and R22 (p-values > 0.05).

As regards the R20 batch, which fill formula did not contained Vitamin C in any form, 0 % of broken capsules were obtained at the different sampling times tested. Thus, formula R20 is not fragile after 12 months from the manufacturing date.

Regarding to the fragility results for the formulas R21 and R22, which fill formula, contained Vitamin C in the form of Calcium ascorbate, no significant differences were observed over time. Both products did not show fragility issues after 12 months from the manufacturing date.

Consequently, the Vitamin C as Ascorbic acid is involved in the root cause of fragility of multivitamin / multimineral products designed (STD 1 and STD2). The use of Vitamin C as Calcium ascorbate allows including Vitamin C in the multivitamin / multimineral products designed without causing its fragility.

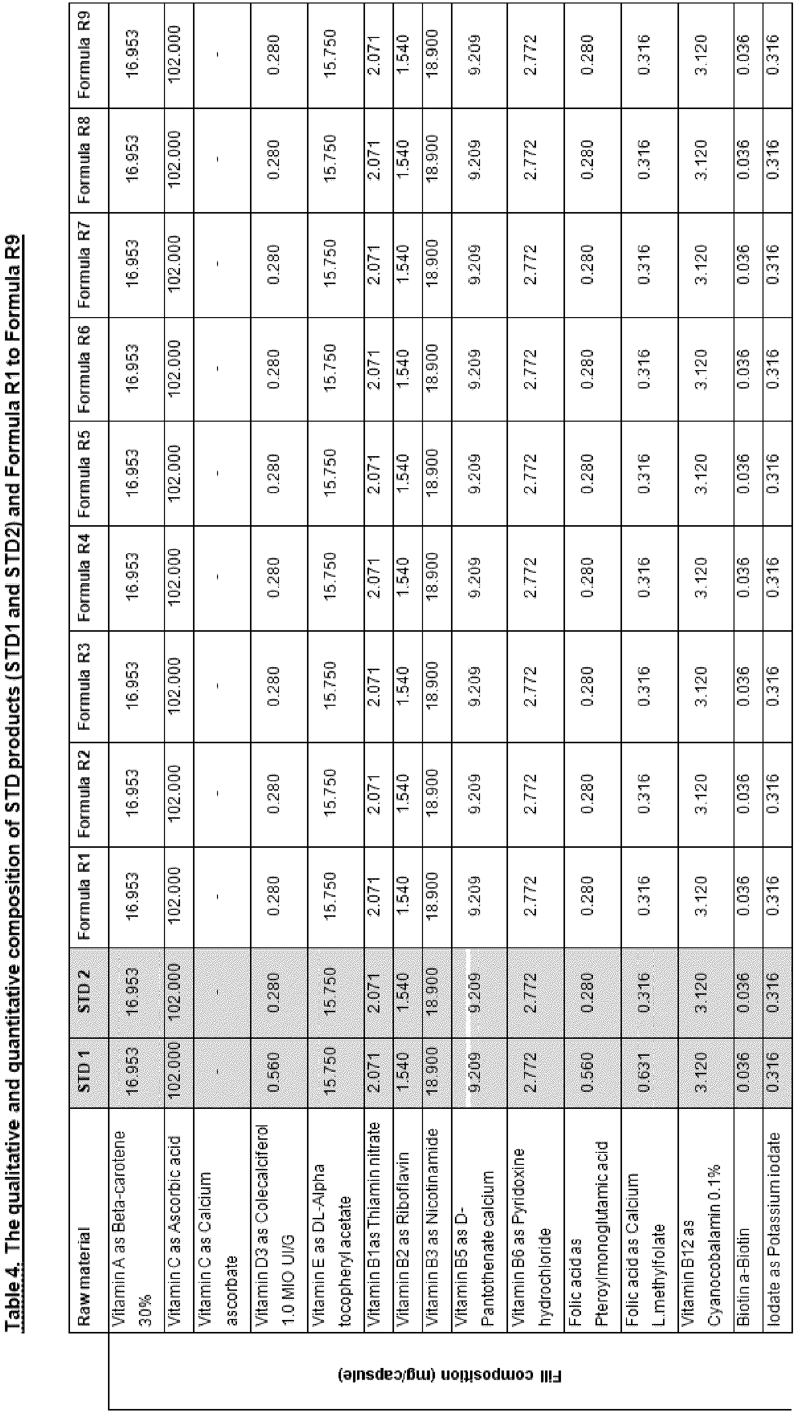

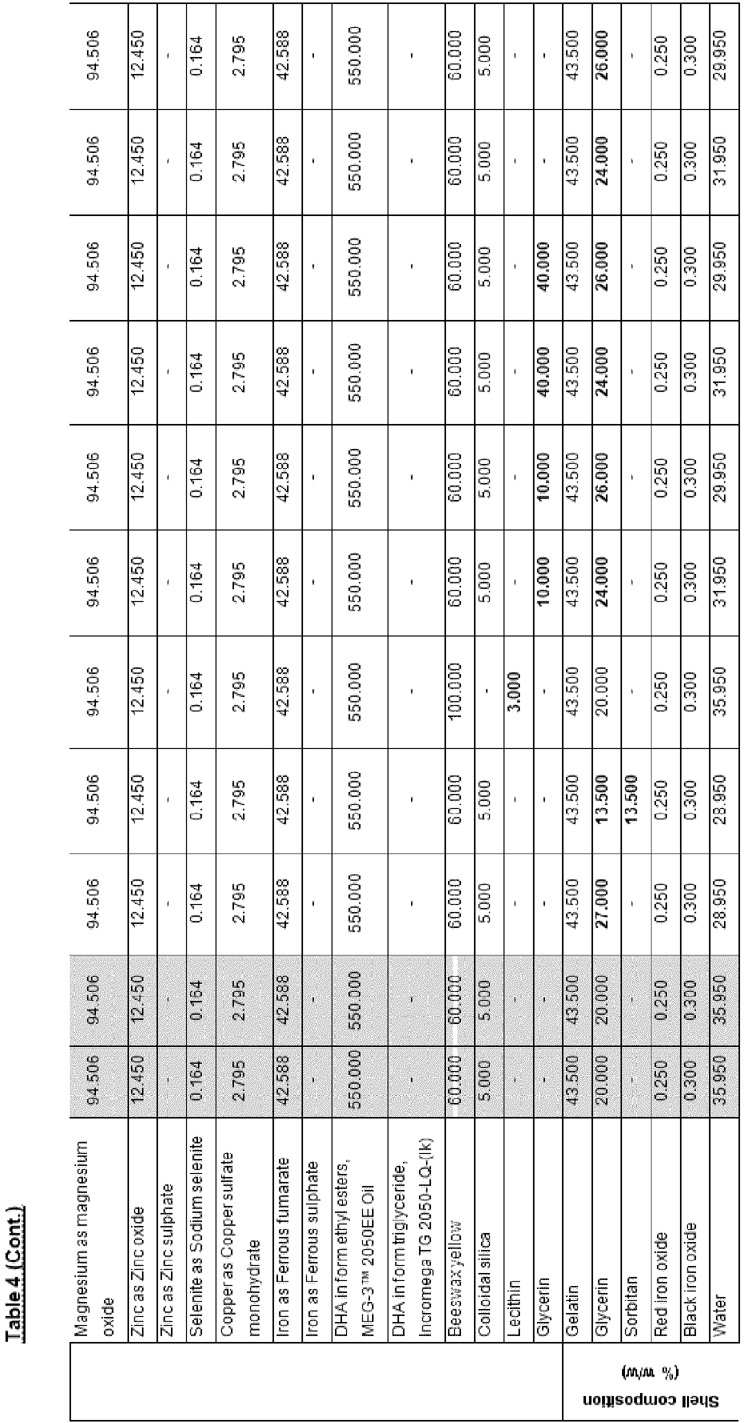

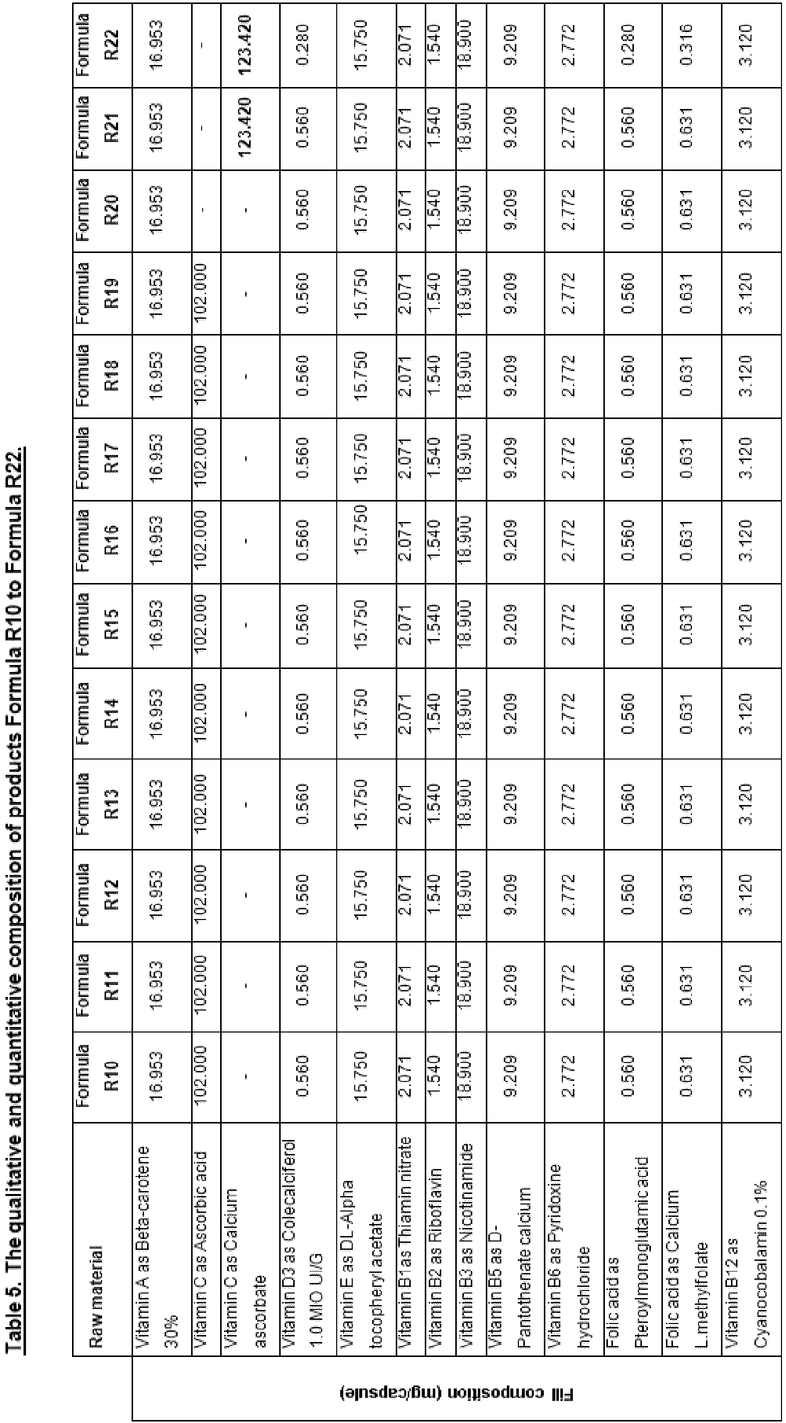

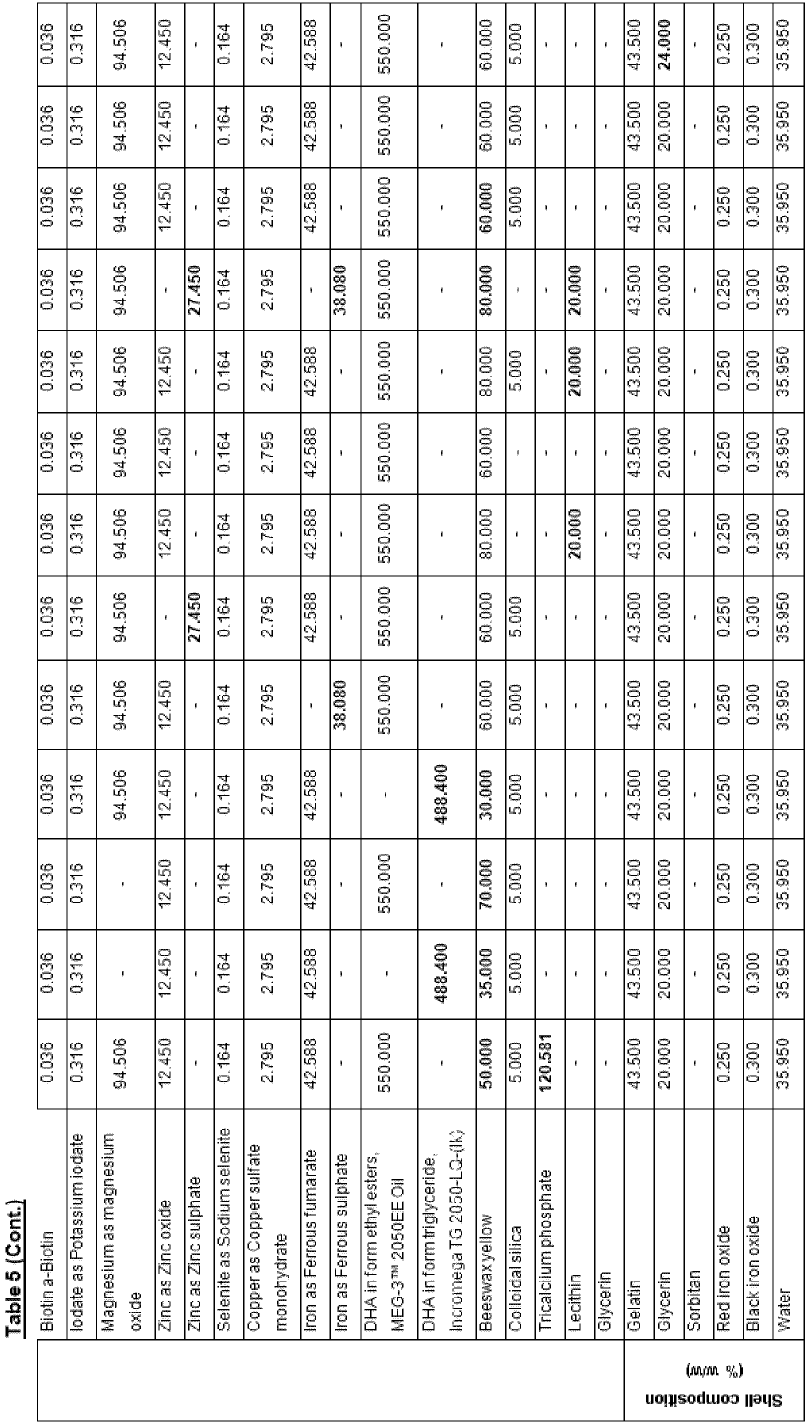

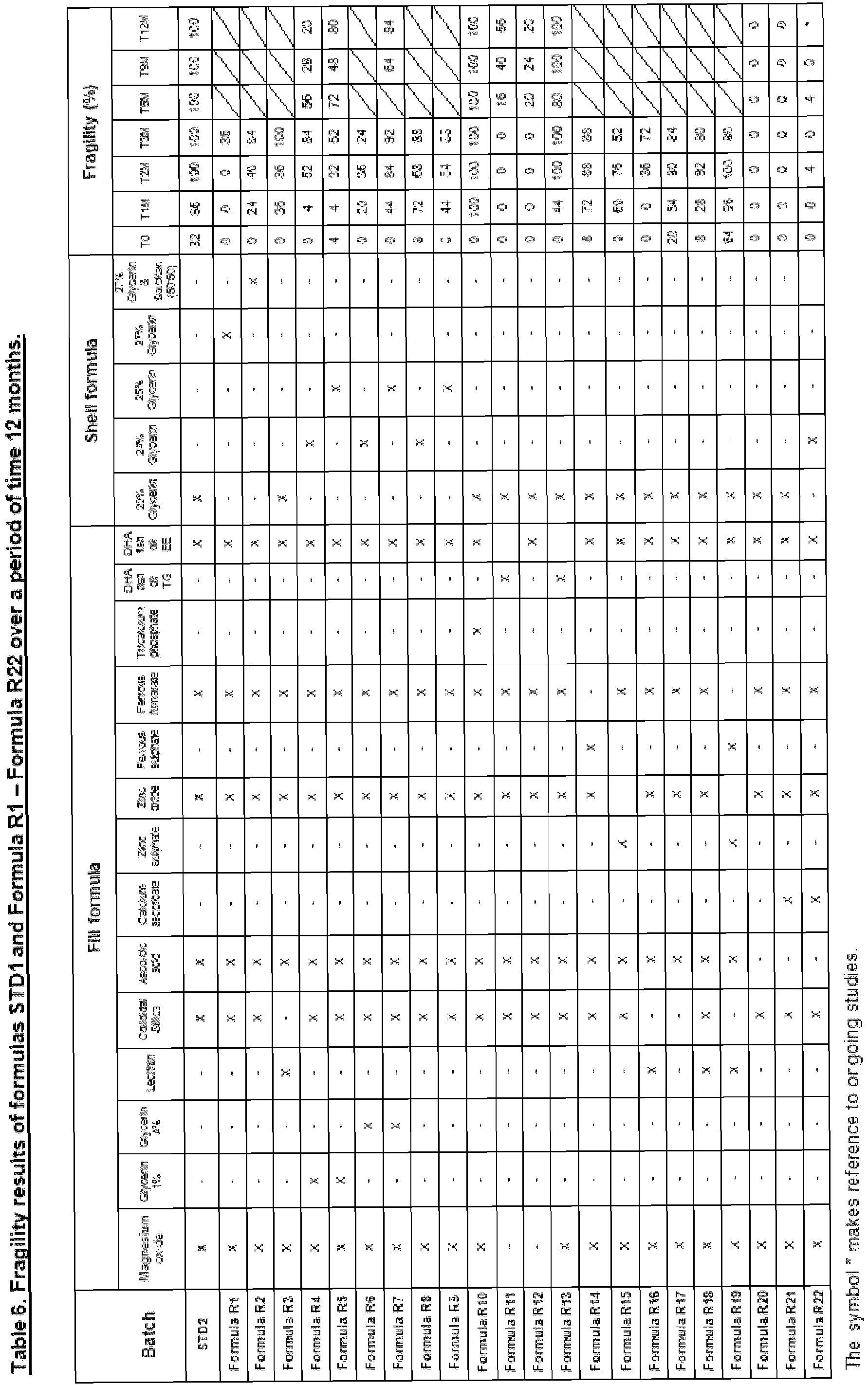

### Example 2. Evaluation of the physical properties over time of new batches G1 and G2.

Two different formulas G1 and G2 were designed to achieve a complete multivitamin/ multimineral prenatal supplement in one softgel (see Table 7). Over time, fragility issues were observed for both of these formulae (see Table 8).

In order to determine the root cause of the fragility for these compositions, the following changes were done manufacturing six new formulations (G3 - G8) included in Table 7.
- Changes in the fill formulas were made:
   - Ascorbic acid to Calcium ascorbate;
   - Copper sulfate to Cupric oxide; and
   - DL-α-Tocopheryl acetate (synthetic Vitamin E) to D-Alpha tocopherol (natural Vitamin E).
- Changes in the shell formulas were also made:
   - Gelatin A to Gelatin B.

In this regard, as described in literature, the type of gelatin -origin, type of denaturing method, molecular weight, bloom strength- can impact on the brittleness capsule. Thus, different gelatin types have been tested.

In order to comply with the specifications about the label claim of each nutrient per capsule and the standard size of the product (22 oblong), the fill weight per capsule was adjusted.

The finished products for each of the above mentioned batches were dried at 22 °C and 20% relative humidity. The drying process was stopped when the flexibility of the capsules reached 7.0 N. The capsules of each of the different batches was packed in polyethylene bags, and stored at warehouse conditions (temperature between 15 - 25 °C).

In order to find the cause/s of brittleness, fragility of the different batches (formulas G1 - G8) was further tested at different times (time zero, 1 month, 2 months 3 months and 6 months). The results were expressed as percentage of broken capsules and are summarized in Table 8 below.

To evaluate the fragility variable of each batch over time, Mood median test was used (95.0 % of CI). The values obtained show that the fragility of different products increases over time significantly (p-values ≤ 0.05) except to formulas G3, G5, G7 and G8 (p-values > 0.05) remaining in values less than 12 % of broken capsules over stability. The common factor of these formulations is the use of Vitamin C such as Calcium ascorbate.

Consequently, the fragility of the multivitamin / multimineral products designed is related to type of Vitamin C. The formulas with Calcium ascorbate were not deemed fragile at time 6 months. However, the formulas with Ascorbic acid were deemed fragile increasing their fragility over time.

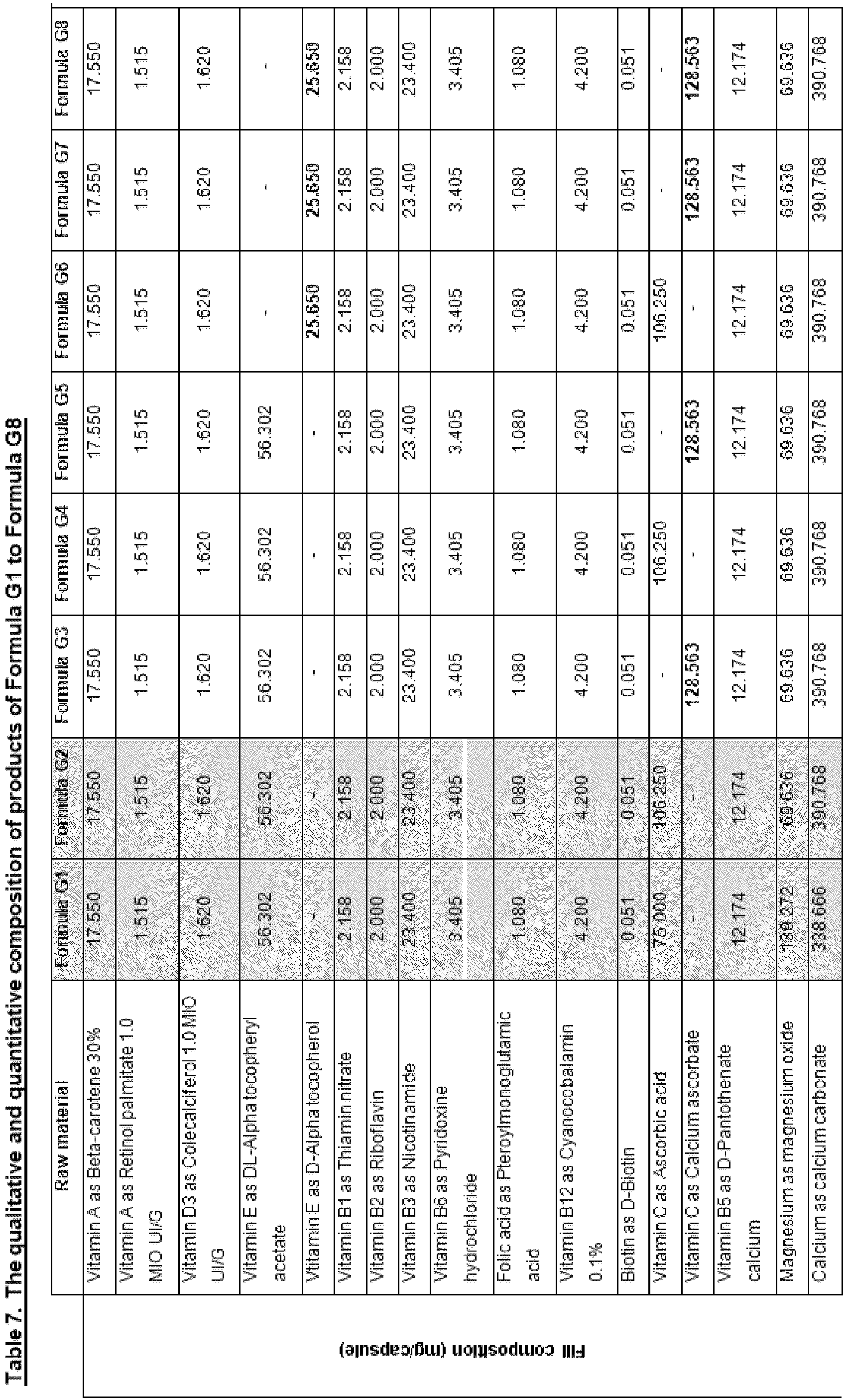

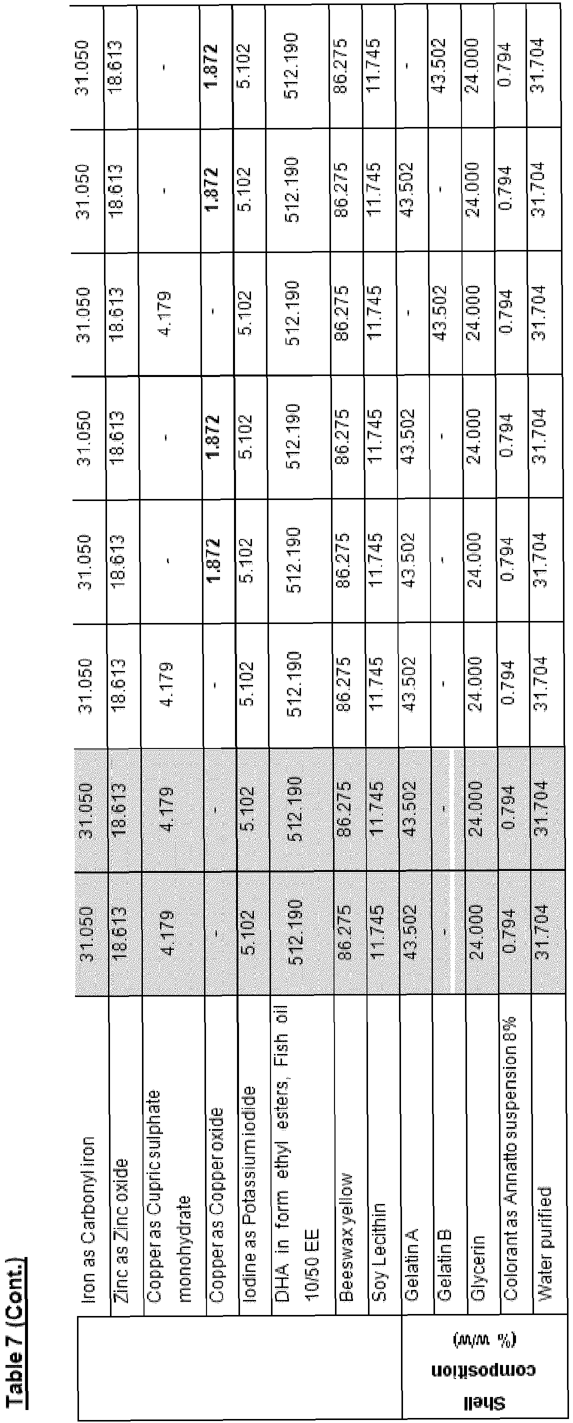

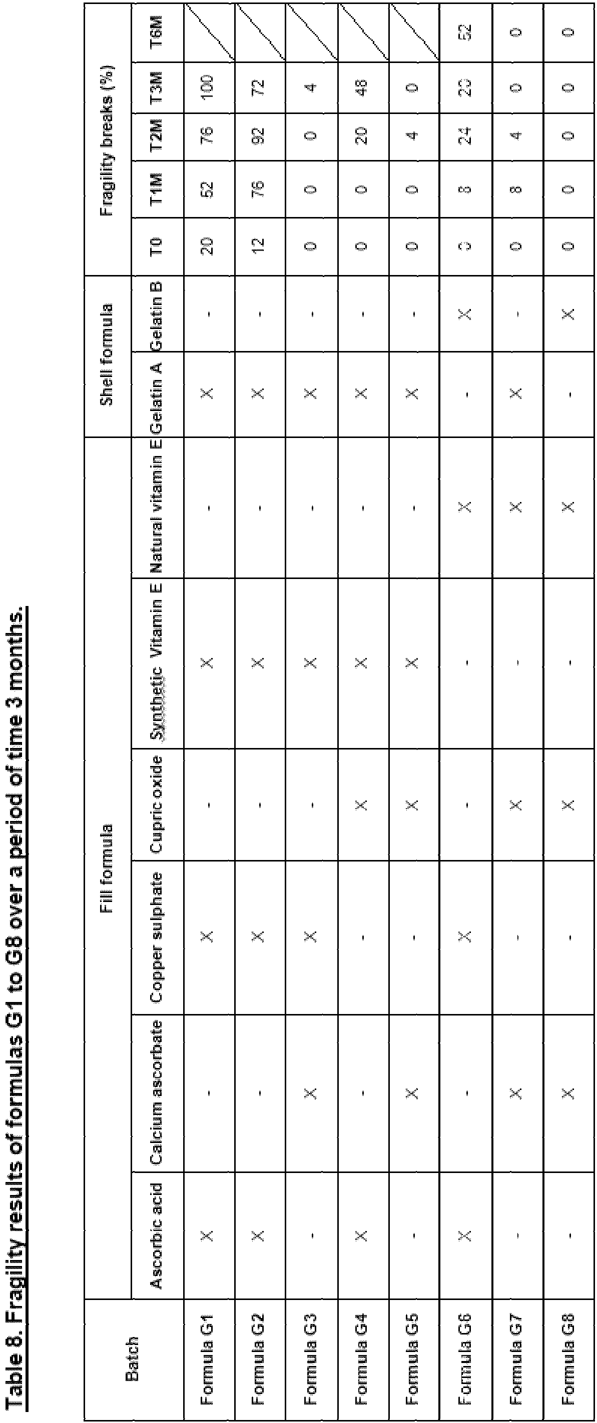

## Claims

1. A method of reducing the fragility of pharmaceutical or dietary compositions in the form of a softgel gelatine capsule comprising (a) one or more vitamin(s), wherein at least one of those vitamin(s) is Vitamin C in the form of Ascorbic acid, and optionally (b) one or more minerals, and pharmaceutically or dietetically suitable carriers (c) such as DHA and/or EPA; wherein the method comprises replacing totally the content of Vitamin C in the form of Ascorbic acid by Vitamin C in the form of Calcium ascorbate or Magnesium ascorbate.

2. The method of claim 1, wherein the pharmaceutical or dietary compositions identified therein, are dietary or pharmaceutical compositions comprising Vitamin C in the form of Ascorbic acid, and at least one further vitamin selected from at least one of the list consisting of: Vitamin A, Vitamin B1, Vitamin B2, Vitamin B3, Vitamin B5, Vitamin B6, Vitamin B9, Vitamin B12, Vitamin H, Vitamin D, Vitamin E, Choline, and Vitamin K, or any combination thereof; and optionally wherein such pharmaceutical or dietary composition further comprises at least one mineral selected from at least one of the list consisting of: Iron, Iodide, Magnesium, Calcium, Zinc (as Zinc oxide or Zinc sulphate), Selenite (as Sodium selenite) and Copper (preferably as Sulphate copper or Copper oxide), or any combination thereof.

3. The method of claim 2, wherein the Vitamin A is Retinol palmitate or β-Carotene.

4. The method of claim 2, wherein the Vitamin B1 is Thiamin or Thiamin mononitrate.

5. The method of claim 2, wherein the Vitamin B5 is Pantothenic Acid or Calcium pantothenate.

6. The method of claim 2, wherein the Vitamin B6 is Pyridoxine or Pyridoxine hydrochloride.

7. The method of claim 2, wherein the Vitamin B9 is Folic acid or Metafolin.

8. The method of claim 2, wherein the Vitamin D is Vitamin D3.

9. The method of claim 2, wherein the Vitamin E is DL-α-Tocopheryl acetate or in the form of natural or synthetic Vitamin E.

10. The method of claim 2, wherein the Choline is Bitartrate choline.

11. The method of claim 2, wherein the Iron is Carbonyl iron, Ferrous fumarate or Ferrous sulphate.

12. The method of claim 2, wherein the Iodide is Potassium iodide or iodate.

13. The method of claim 2, wherein the Zinc is Zinc oxide or Zinc sulphate.

14. The method of claim 2, wherein the Selenite is Sodium selenite.

15. The method of claim 2, wherein the Copper is Sulphate copper or Copper oxide.

## Patentansprüche

1. Verfahren zur Verringerung der Zerbrechlichkeit von pharmazeutischen oder diätetischen Zusammensetzungen in Form einer Softgel-Gelatinekapsel, umfassend
(a) ein oder mehrere Vitamin(e), wobei mindestens eines dieser Vitamine Vitamin C in Form von Ascorbinsäure ist, und gegebenenfalls
(b) einen oder mehrere Mineralstoffe, und pharmazeutisch oder diätetisch geeignete Trägerstoffe
(c) wie z.B. DHA und/oder EPA;
wobei das Verfahren das vollständige Ersetzen des Gehalts an Vitamin C in Form von Ascorbinsäure durch Vitamin C in Form von Calciumascorbat oder Magnesiumascorbat umfasst.

2. Verfahren nach Anspruch 1, wobei die darin identifizierten pharmazeutischen oder diätetischen Zusammensetzungen diätetische oder pharmazeutische Zusammensetzungen sind, die Vitamin C in Form von Ascorbinsäure und mindestens ein weiteres Vitamin umfassen, das aus der Liste bestehend aus: Vitamin A, Vitamin B1, Vitamin B2, Vitamin B3, Vitamin B5, Vitamin B6, Vitamin B9, Vitamin B12, Vitamin H, Vitamin D, Vitamin E, Cholin und Vitamin K oder eine beliebige Kombination davon ausgewählt ist; und wobei eine solche pharmazeutische oder diätetische Zusammensetzung gegebenenfalls außerdem mindestens einen Mineralstoff umfasst, der aus der Liste bestehend aus: Eisen, Jodid, Magnesium, Kalzium, Zink (als Zinkoxid oder Zinksulfat), Selenit (als Natriumselenit) und Kupfer (vorzugsweise als Kupfersulfat oder Kupferoxid), oder eine beliebige Kombination davon ausgewählt ist.

3. Verfahren nach Anspruch 2, wobei das Vitamin A Retinolpalmitat oder β-Carotin ist.

4. Verfahren nach Anspruch 2, wobei das Vitamin B1 Thiamin oder Thiaminmononitrat ist.

5. Verfahren nach Anspruch 2, wobei das Vitamin B5 Pantothensäure oder Calciumpantothenat ist.

6. Verfahren nach Anspruch 2, wobei das Vitamin B6 Pyridoxin oder Pyridoxinhydrochlorid ist.

7. Verfahren nach Anspruch 2, wobei das Vitamin B9 Folsäure oder Metafolin ist.

8. Verfahren nach Anspruch 2, wobei das Vitamin D Vitamin D3 ist.

9. Verfahren nach Anspruch 2, wobei das Vitamin E DL-α-Tocopherylacetat oder in Form von natürlichem oder synthetischem Vitamin E ist.

10. Verfahren nach Anspruch 2, wobei das Cholin Bitartrat-Cholin ist.

11. Verfahren nach Anspruch 2, wobei das Eisen Carbonyleisen, Eisenfumarat oder Eisensulfat ist.

12. Verfahren nach Anspruch 2, wobei das Iodid Kaliumiodid oder Iodat ist.

13. Verfahren nach Anspruch 2, wobei das Zink Zinkoxid oder Zinksulfat ist.

14. Verfahren nach Anspruch 2, wobei das Selenit Natriumselenit ist.

15. Verfahren nach Anspruch 2, wobei das Kupfer Kupfersulfat oder Kupferoxid ist.

## Revendications

1. Méthode de réduction de la fragilité des compositions pharmaceutiques ou diététiques sous forme de capsule de gélatine molle comprenant
(a) une ou plusieurs vitamines, caractérisée(s) en ce que au moins une de ces vitamines est la vitamine C sous forme d'acide ascorbique, et éventuellement
(b) un ou plusieurs minéraux, et des supports pharmaceutiquement ou diététiquement appropriés
(c) tels que le DHA et/ou l'EPA ;
**caractérisée en ce que** la méthode comprend le remplacement total du contenu de la vitamine C sous forme d'acide ascorbique par de la vitamine C sous forme d'ascorbate de calcium ou d'ascorbate de magnésium.

2. La méthode de la revendication 1, dans laquelle les compositions pharmaceutiques ou diététiques identifiées, sont des compositions diététiques ou pharmaceutiques comprenant de la vitamine C sous forme d'acide ascorbique, et au moins une autre vitamine choisie parmi au moins une de la liste constituée de : Vitamine A, Vitamine B1, Vitamine B2, Vitamine B3, Vitamine B5, Vitamine B6, Vitamine B9, Vitamine B12, Vitamine H, Vitamine D, Vitamine E, Choline, et Vitamine K, ou toute combinaison de celles-ci ; et éventuellement en ce que cette composition pharmaceutique ou diététique comprend en outre au moins un minéral choisi parmi au moins une de la liste constituée de : Fer, Iodure, Magnésium, Calcium, Zinc (sous forme d'oxyde de zinc ou de sulfate de zinc), Sélénite (sous forme de sélénite de sodium) et Cuivre (de préférence sous forme de sulfate de cuivre ou d'oxyde de cuivre), ou toute combinaison de ceux-ci.

3. La méthode de la revendication 2, dans laquelle la vitamine A est le palmitate de rétinol ou le β-carotène.

4. La méthode de la revendication 2, dans laquelle la vitamine B1 est la thiamine ou le mononitrate de thiamine.

5. La méthode de la revendication 2, dans laquelle la vitamine B5 est l'acide pantothénique ou le pantothénate de calcium.

6. La méthode de la revendication 2, dans laquelle la vitamine B6 est la pyridoxine ou le chlorhydrate de pyridoxine.

7. La méthode de la revendication 2, dans laquelle la vitamine B9 est l'acide folique ou la métafoline.

8. La méthode de la revendication 2, dans laquelle la vitamine D est la vitamine D3.

9. La méthode de la revendication 2, dans laquelle la vitamine E est l'acétate de DL-α-Tocophéryl ou sous forme de vitamine E naturelle ou synthétique.

10. La méthode de la revendication 2, dans laquelle la choline est du bitartrate de choline.

11. La méthode de la revendication 2, dans laquelle le fer est du fer carbonique, du fumarate ferreux ou du sulfate ferreux.

12. La méthode de la revendication 2, dans laquelle l'iodure est l'iodure ou l'iodate de potassium.

13. La méthode de la revendication 2, dans laquelle le zinc est de l'oxyde de zinc ou du sulfate de zinc.

14. La méthode de la revendication 2, dans laquelle la sélénite est la sélénite de sodium.

15. La méthode de la revendication 2, dans laquelle le cuivre est du cuivre sulfaté ou de l'oxyde de cuivre.
